# EUROPEAN PATENT APPLICATION

(11) **EP 4 197 566 A1**
(43) Date of publication of application: **21.06.2023**
(21) Application number: 21215841.4
(22) Date of filing: 20.12.2021
(51) Int. Cl.: A61M 1/06, A61M 1/00

(54) **BREAST PUMP SYSTEM**

(71) Applicant: Koninklijke Philips N.V., 5656 AG Eindhoven (NL)
(72) Inventor: LIPSCH, Job, Eindhoven (NL); VAN DER KOOI, Johannes Tseard, Eindhoven (NL)
(74) Representative: Philips Intellectual Property & Standards

(57) **Abstract**

A breast pump system comprises a first kit, a second kit or a buffer, and a pump for delivering a negative pressure (i.e. a pressure lower than the ambient atmospheric pressure). This system enables pressure to be equalized between the first kit and the second kit or buffer, thus reducing energy loss by venting a stored pressure to the ambient pressure.

## Description

### FIELD OF THE INVENTION

This invention relates to breast pump systems, for example with an expression kit for each breast.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted. The breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

Often, it is desirable to express both breasts at the same time. A user will then typically acquire a set of identical devices (e.g. in one housing) to achieve a setup which performs stimulation and expression independently for each breast.

This results in the use of two pumps, which is costly both in terms of components as well as power consumption, and it may also result in a high level of noise.

US 2020/155738 discloses a wearable breast pump. In one example, there are two expression kits, but only one has the controller or battery or pump to be used by both expression kits. This saves cost in terms of the number of components, but the issue of power consumption is not addressed.

WO 2020/007671 discloses a double breast pump device in which enables vacuum to be transferred between two expression kits. Thus, the volume of one expression kit is used as a storage volume of under pressure for the other expression kit. However, a complicated valve arrangement is needed.

### SUMMARY OF THE INVENTION

The invention is defined by the claims.

According to examples in accordance with an aspect of the invention, there is provided a breast pump system comprising:
a first kit; and
a second kit or a buffer,
a pump for delivering a negative pressure;
a valve arrangement which selectively provides an interconnection between the pump, the ambient pressure, the first kit and the second kit or buffer; and
a controller for controlling the valve arrangement,
wherein the valve arrangement comprises:
   a first valve unit connected between the first kit and the pump and a second valve unit connected between the second kit or buffer and the pump or the first valve unit, wherein the first and second valve units are controllable to connect the first kit to a selected one of the pump, the ambient pressure and the second kit or buffer,
   and wherein the first and second valve units each comprise a single valve actuator.

This system reduces cost by enabling only a single pump to be used, and it additionally reduces energy consumption by enabling the first kit and the second kit or buffer to be coupled together, so that instead of venting pressure to the atmosphere, the pressure is reused. When two kits are used, they may be controlled in an alternating sequence. The system reduces the load on the pump by reusing the vacuum. This reduced load translates to power savings.

The system only needs two valve actuators (e.g. solenoids) to provide the require pressure sharing function.

The invention may be implemented as a system with two expression kits or with one kit and a buffer specifically provided for the purpose of reducing energy loss caused by venting pressure to the ambient surroundings.

For examples using a buffer, there is no need to use the pump to apply pressure to the buffer. For example using two kits, each kit is provided with an actively pumped pressure profile using the pump.

In one example, the second kit or buffer comprises a buffer, the first valve unit comprises a three-port valve which couples the first kit, the ambient pressure and the pump, and the second valve unit couples the buffer to the pump side of the first valve unit first kit or to a pressure stop.

In another example, the second kit or buffer comprises a buffer, the first valve unit comprises a three-port valve which couples the first kit, the ambient pressure and the pump, and the second valve unit couples buffer to the kit side of the first valve unit or to a pressure stop.

These are two simple implementations of the kit with a buffer. Each example may use a three port valve and a two port valve. A pressure stop (i.e. a valve connection which only serves to retain the pressure level in the buffer) is used to hold the pressure in the buffer until the pressure is to be equalized with the first kit. The buffer may connect directly to the expression kit through the second valve unit, or it may connect to the expression through both valve units in series (for example via a pump port).

In another example, the second kit or buffer again comprises a buffer, the first valve unit comprises a pair of three-port valves which couple the first kit, the ambient pressure, the buffer (in combination with the second valve unit) and the pump, and the second valve unit comprises a pair of three port valves which couple the buffer, the first kit, the pump and a pressure stop.

In this case, each valve unit comprises a pair of three-port valves. This enables a simpler control sequence and provides an easier way to interchange the buffer for a second kit.

In another example, the second kit or buffer comprises a second kit, and the first and second valve units each comprise a pair of three-port valves coupled together, thereby having four external ports.

The, or each, kit for example comprises a breast shield, and typically also a membrane between the pump and the breast to prevent milk contaminating the pump.

When a vacuum level is vented from one kit (i.e. the pressure is being increased at the end of a vacuum phase), rather than simply venting the kit to the ambient surroundings, the negative pressure is used for the vacuum cycle in the other kit or else it is stored in a buffer to be reused by the same kit. This saves power in that the work done by the pump is reduced.

The controller is for example adapted to equalize a pressure level between the first kit and the second kit or buffer at, or near, the end of the pressure cycle for the kit. This can be achieved by controlling the valve arrangement to open a fluid coupling between them. The negative pressure level at the end of a cycle for one kit is used as a negative pressure source for the other kit, next in the alternating sequence, or is stored in a buffer. Connecting the two kits (or kit and buffer) doubles the volume so that the stored pressure contributes half of the required pressure in the case of similar volumes.

The valve arrangement provides a simple and low cost way to control the fluid connections. Each kit is associated with a respective valve unit. By coupling the valve units together, the pressure equalizing mentioned above is implemented. By using a pair of three-port valves coupled together for each valve unit, a simple and low cost way is provided to implement a valve unit with four external ports (for example, the case of two kits, one to the kit, one to the other kit, one to the ambient pressure, and one to the pump).

In a system with two kits, the controller is for example adapted to control the valve arrangement to couple:
the first kit to the pump and the second kit to the ambient pressure in a first configuration; and
the first kit and second kit together in a second configuration to equalize a pressure level between the first kit and second kit;
the second kit to the pump and the first kit to the ambient pressure in a third configuration.

Thus, one kit has a negative pressure applied while the other kit is vented. This enables an alternating sequence to be used.

The controller for example comprises a cyclic pump waveform generator for controlling the pump and for controlling the valve arrangement. There may be a single waveform generator for both control signals or separate waveform generators.

The cyclic waveform generator for example generates a cyclic pump waveform and a cyclic valve waveform, thereby to implement a cycle with four phases in sequence:
a first phase comprising the first configuration and with the pump generating a negative pressure;
a second phase comprising the second configuration;
a third phase comprising the third configuration and with the pump generating a negative pressure;
a fourth phase comprising the second configuration.

These four phases together define cyclic pressure waveforms for both kits. A decreasing pressure (i.e. increasing vacuum level) in the cycle for one kit coincides with an increasing pressure (i.e. decreasing vacuum level) in the cycle for the other kit. Energy is saved during the second and fourth phases, when an increase in pressure in one kit is balanced by a reduced in pressure in the other kit, rather than simply being a loss to the ambient pressure of the surroundings.

The pump is for example turned off in the second and fourth phases to save power. However, the pump may be kept running at low power with a buffer being used, for example for noise reduction.

The breast pump system for example comprises a first actuator for actuating a first three-port valve of the first valve unit and a first three-port valve of the second valve unit, and a second actuator for actuating a second three-port valve of the first valve unit and a second three-port valve of the second valve unit.

The two actuators e.g. solenoids are shared in a particular way between the two valve units.

In one particular example:
the first three-port valve of the first valve unit has a connection to the first kit, a connection to the first three-port valve of the second valve unit and a connection to the second three-port valve of the first valve unit;
the second three-port valve of the first valve unit has a connection to the ambient pressure, a connection to the pump and a connection to the first three-port valve of the first valve unit;
the first three-port valve of the second valve unit has a connection to the second kit, a connection to the first three-port valve of the first valve unit and a connection to the second three-port valve of the second valve unit; and
the second three-port valve of the second valve unit has a connection to the ambient pressure, a connection to the pump and a connection to the first three-port valve of the second valve unit.

This provides one particular valve actuation arrangement with only two actuators.

Each kit for example comprises a suction cup, comprising two air chambers separated by a flexible movable membrane. One chamber is defined in part by the breast and the other is isolated from the breast. This avoids contact between the pump and expressed milk. The cyclic waveform for each kit is for example an expression waveform. However, the same approach may be applied to a stimulation waveform.

The invention also provides a method of controlling a breast pump system for the non-therapeutic expression of milk, wherein the breast pump system has a first kit, a second kit or a buffer, a pump for delivering a negative pressure and a valve arrangement which selectively provides an interconnection between the pump, the ambient pressure, the first kit and the second kit or buffer,
wherein the method comprises:
controlling a single pump to generate a negative pressure;
controlling the single pump and the valve arrangement to apply a negative pressure to the first kit and equalize a pressure level between the first kit and second kit or buffer, by:
   controlling a first valve unit connected between the first kit and the pump and a second valve unit connected between the second kit or buffer and the pump or the first valve unit,
   wherein the first and second valve units are controlled to connect the first kit to a selected one of the pump, the ambient pressure and the second kit or buffer,
   and wherein the method comprises controlling each of the first and second valve units with a single respective valve actuator.

The method may comprise controlling the pump to generate the negative pressure with a cyclic pump waveform and controlling the valve arrangement to apply the negative pressure to the first kit and to a second kit in an alternating sequence using a cyclic valve waveform.

For the method applied to two expression kits, a cycle may be implemented with four phases in sequence:
a first phase with a negative pressure coupled to the first kit and the second kit vented to the ambient pressure;
a second phase with the first kit and second kit coupled together
a third phase with a negative pressure coupled to the second kit and the first kit vented to the ambient pressure; and
a fourth phase with the first kit and second kit coupled together.

The pump may be turned off in the second and fourth phases. The valve arrangement is preferably controlled using exactly two valve actuators.

The method may comprise using a first valve actuator to control a first three-port valve of the first valve unit and a first three-port valve of the second valve unit, and using a second valve actuator to control a second three-port valve of the first valve unit and a second three-port valve of the second valve unit.

The invention also provides a computer program comprising computer program code means which is adapted, when said program is run on a controller of a breast pump system, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a mother wearing two wearable breast pumps;
Figure 3 shows a breast pump system having a pump shared between two expression kits;
Figures 4A to 4D show an example of an arrangement of a breast pump system at four different phases of an overall pressure cycle for two expression kits;
Figure 5 shows the cavity pressure in the two kits over time;
Figure 6 shows an example of an implementation of each valve unit and shows the first valve unit by way of example;
Figure 7 shows control signals for the valves and a pump control signal;
Figure 8 shows the mechanical power over time for a conventional use of a pair of independent expression kits and for the approach of the invention;
Figure 9 shows the cavity pressure over time for the expression kits based on a simulation as well as the pump pressure;
Figures 10 to 12 show one detailed implementation of the valve arrangement and its control actuators for two expression kits in first to third configurations;
Figures 13 to 18 show a first detailed implementation of the valve arrangement and its control actuators for an expression kit and a buffer in first to fifth configurations;
Figures 19 to 24 show a second detailed implementation of the valve arrangement and its control actuators for an expression kit and a buffer in first to sixth configurations.
Figures 25 to 30 show a third detailed implementation of the valve arrangement and its control actuators for an expression kit and a buffer in first to sixth configurations.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system which comprises a first kit, a second kit or a buffer, and a pump for delivering a negative pressure (i.e. a pressure lower than the ambient atmospheric pressure). This system enables pressure to be equalized between the first kit and the second kit or buffer, thus reducing energy loss because a built-up pressure is not vented to the ambient pressure.

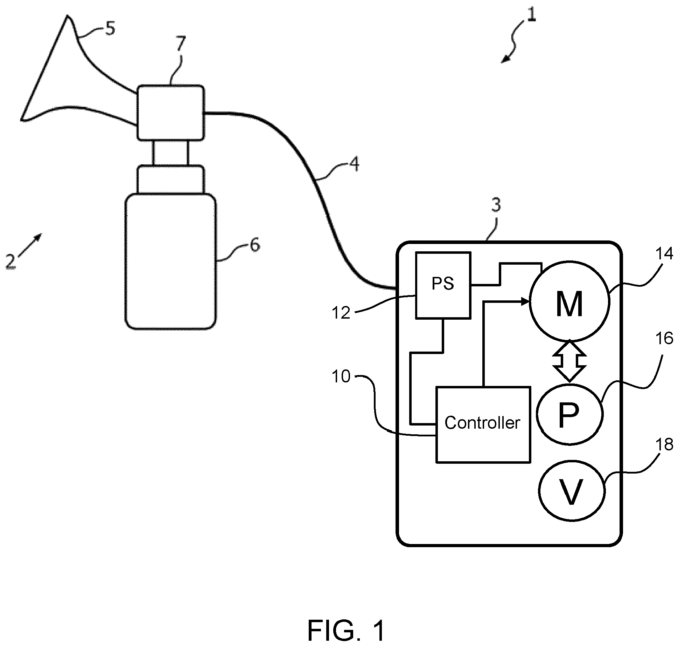

Figure 1 shows a known breast pump system 1, comprising a single expression kit 2 and a pump arrangement 3 which are connected via a tube 4. The pump arrangement includes various components in addition to the pump, so may be considered to be a general operating unit.

The expression kit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The vacuum pump comprises an impeller driven by the motor. The controller controls 10 the operation of the power source 12 and motor 14 (and hence the vacuum pump 16). The pump arrangement 3 further comprises a vacuum venting component, such as a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast.

The vacuum is applied to the breast at intervals. That is, a pressure differential is applied on a cyclic basis. In this known system, after a vacuum has been established, the pressure from the vacuum is vented by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression kit which enables milk to be expressed from the breast of a user. When the solenoid valve is opened, the vacuum generated by the vacuum pump is vented as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. In one type of design, a top part contains the drivetrain (pump, motor, controller) and a bottom part forms the collection vessel. This enables breast pumping to be performed more discretely. Of course, other designs are possible.

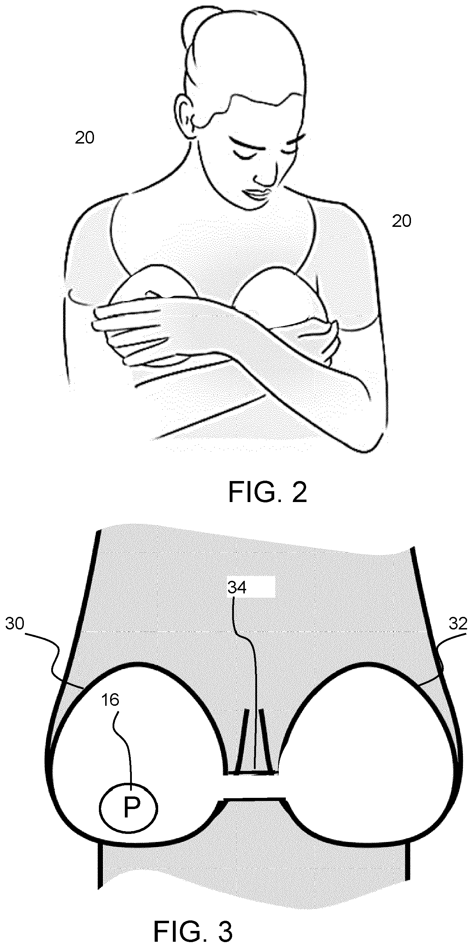

Figure 2 shows a mother wearing two wearable breast pumps 20.

In such a design, the funnel 5 of Figure 1 is implemented as breast shield, and this breast shield is received within an outer enclosure of the wearable breast pump.

The invention will be described with reference to a breast pump system having two expression kits. Each expression kit is an assembly for fitting over the breast of a user, and for example includes a breast shield and optionally also a membrane (known as the diaphragm) between the breast shield and the breast, so that the membrane limits the parts of the breast pump system that can be contaminated with milk.

The invention may however also be applied to breast pump systems with a single expression kit. The invention relates in particular to reducing energy loss when venting an expression kit to the ambient surroundings. When two expression kits are present, the invention involves equalizing pressure between them so that the pressure that would be vented can be considered to be re-used. However, a buffer (i.e. pressure storage vessel) may be used instead of the second expression kit to store energy as a vacuum rather than allowing it to be vented to the ambient pressure.

An example of the invention as applied to a breast pump system with two expression kits will first be described. Examples will then be provided to show how the invention may be applied to a system with one expression kit and a buffer.

Figure 3 shows a breast pump system comprising a first expression kit 30 and a second expression kit 32. Each expression kit is a wearable device, and each expression kit includes a breast shield. A single pump is provided for operating the two expression kits. In the example shown, the pump is located at one expression kit and a coupling 34 is provided between the expression kits so that the single pump may deliver an under pressure to both expression kits. The expression kits may each comprise a milk sensor for sensing milk flow and for measuring flow volume.

In a typical arrangement, the expression kits are independent and identical. However, as shown in Figure 3 it is also possible for expression kits to have different sets of components to reduce the duplication of hardware. For example, US 2020/155738 discloses a wearable breast pump with two expression kits, but only one has the controller or battery or pump to be used by both expression kits.

The preferred implementation of the invention relates to a design with a shared pump which is used by two expression kits as represented in Figure 3. The overall system may be wearable (so that the pump is located at one of the expression kits) or it may be portable, for example with the pump 16 remote from the expression kits, for example mounted on a belt. When one expression kit has the pump, other components may be at the other kit, such as a controller or battery or valves, to balance the weight and size/shape of the two expression kits.

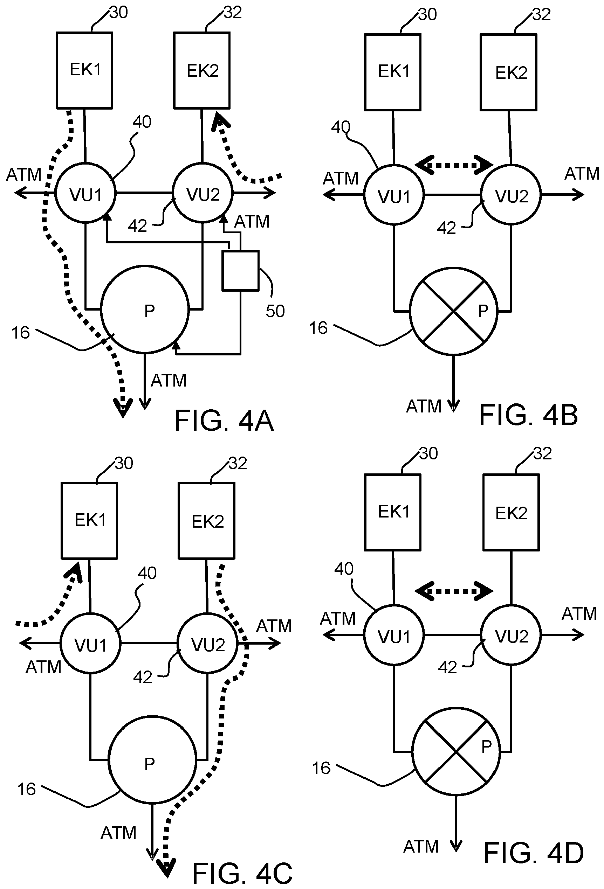

Figures 4A to 4D show an example of the arrangement of the invention (in schematic form) at four different phases of an overall pressure cycle for the two expression kits. First and second expression kits 30, 32 (EK1, EK2) are shown. Each expression kit defines a cavity to which a negative pressure may be applied. A valve arrangement is provided which selectively provides an interconnection between the pump, the ambient pressure, and the first and second kits (or between the first kit and a buffer in a more general example). A controller 50 is provided for controlling the valve arrangement and the pump. The controller is shown only in Figure 4A for simplicity.

The valve arrangement comprises a first valve unit 40 connected between the first kit 30 and the pump 16 and a second valve unit 42 connected between the second kit 32 and the pump 16. The first valve unit 40 is controllable to connect the first kit to a selected one of the pump, the ambient pressure and the second kit. Thus, it has four external ports. The second valve unit 42 is controllable to connect the second kit to a selected one of the pump, the ambient pressure and the first kit. Thus, it also has four external ports.

As explained above, each expression kit may define a first cavity over the breast of the user, and a second cavity which is separated from the first cavity by membrane (diaphragm). In such a case, the second cavity of each expression kit connects to a respective valve unit 40, 42 (VU1, VU2).

For the avoidance of doubt, the "connections" discussed in this disclosure are fluid couplings, i.e. couplings which allow airflow to be transported through them, resulting in equalization of pressure.

One port of each valve unit 40, 42 is connected to the corresponding port of the other valve unit. Thus, the combination of the two valve units 40, 42 defines a valve arrangement. The valve arrangement is for controllably coupling the first and second kits together, or for coupling a selected one of the kits to the pump, or for coupling a selected one of the kits to ambient pressure. The two connections to the pump 16 may in fact be combined into a single connection, in that the pump delivers an under pressure (relative to the ambient pressure) to an outlet port, and the two valve units each connect to that same outlet port.

The overall valve arrangement thus needs four external ports; atmospheric pressure (assuming both atmospheric pressure connections can be assumed to be connected as a single port), the pump and the first and second kits).

The coupling between the two valve units 40, 42 is controllable by the setting of the valve arrangement, in that it may be opened and closed. By coupling the valve units together, pressure equalizing between the cavities of the two expression kits is implemented.

The controller 50 is for controlling the pump 16 and the valve arrangement 40, 42. In particular it applies the negative pressure generated by the pump 16 to the first and second kits 30, 32 in an alternating sequence.

This system reduces cost by enabling only a single pump to be used, but it additionally reduces energy consumption by controlling the two kits in an alternating sequence. This reduces the load on the pump at any time as a result of the re-use of vacuum.

Figures 4A to 4D show four phases of an overall pressure cycle.

Figure 4A shows the first phase. In this phase, as shown by the dotted arrows, the first kit 30 is coupled to the pump, the pump generates a negative pressure, and the second kit 32 is vented to the ambient pressure ATM.

Figure 4B shows the second phase. In this phase, as shown by the dotted arrows, the first and second kits 30, 32 are coupled together. Optionally, the pump is turned off, but equally it may be applying a low pressure. For example, continuous running of the pump at a lower speed reduces the noise levels or irritation by creating a continuous sound instead of alternating sound bursts.

Figure 4C shows the third phase. In this phase, as shown by the dotted arrows, the second kit 32 is coupled to the pump, the pump generates a negative pressure, and the first kit 30 is vented to the ambient pressure ATM.

Figure 4D shows the fourth phase. In this phase, as again shown by the dotted arrows, the first and second kits 30, 32 are coupled together and the pump is again optionally turned off.

These four phases together define cyclic pressure waveforms for both kits.

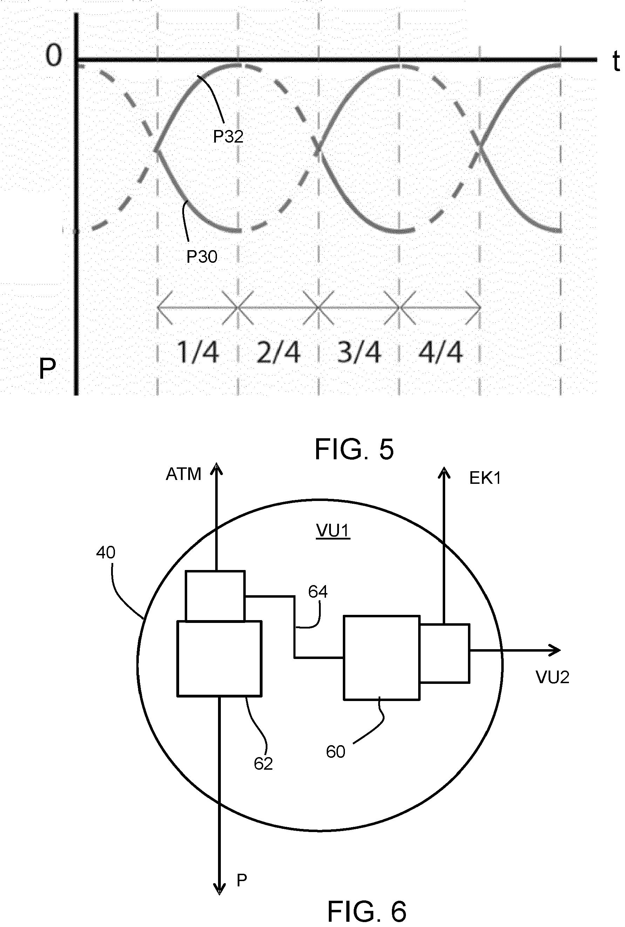

Figure 5 shows the cavity pressure in the two kits 30, 32 over time. The first kit has cavity pressure P30 and the second kit has pressure P32.

The four phases are shown in Figure 5 as 1/4, 2/4, 3/4 and 4/4.

During the first phase, the pressure P30 is dropping (to a deeper vacuum level) because the first kit 30 is connected to the pump and the pump is turned on. The pressure P32 is increasing because the second kit is vented to the ambient pressure.

During the second phase, the pump is turned off, and the two pressures P30 and P32 are equalizing. Thus, the increase of pressure P30 is not a loss to the ambient surroundings, but contributes to the reduction in pressure P32. This initial reduction in pressure P32 from the maximum (ambient) pressure thus does not need to be generated by the pump.

During the third phase, the pressure P32 is dropping (to a deeper vacuum level) because the second kit 32 is connected to the pump and the pump is turned on. The pressure P30 is increasing because the first kit is vented to the ambient pressure.

During the fourth phase, the pump is turned off, and the two pressures P30 and P32 are equalizing. Thus, the increase of pressure P32 is not a loss to the ambient surroundings, but contributes to the reduction in pressure P30. This initial reduction in pressure P30 from the maximum (ambient) pressure again does not need to be generated by the pump.

A decreasing pressure (i.e. increasing vacuum level) in the cycle for one kit can be seen to coincide with an increasing pressure (i.e. decreasing vacuum level) in the cycle for the other kit. Energy is saved in particular during the second and fourth phases, when an increase in pressure in one kit is balanced by a reduced in pressure in the other kit, rather than simply being provided by ambient pressure of the surroundings.

It can be seen that the control system equalizes the pressure level between the first and second kits at the end of the pressure cycle for each kit, i.e. when the pressure has reached a minimum level (i.e. a maximum negative level).

Figure 6 shows an example of an implementation of each valve unit 40, 42, and shows the first valve unit 40 by way of example.

The valve unit comprises a pair of three-port valves 60, 62 coupled together, thereby having four external ports. This is a simple way to implement a valve unit with four external ports (one to the kit EK1, one to the valve unit VU2 of the other kit, one to the ambient pressure ATM, and one to the pump P).

As is explained in more detail below, this arrangement also enables the overall valve arrangement to be controlled with only two actuators e.g. solenoids.

The first valve 60 is a coupling valve for controlling the coupling of the expression kit to either the other valve unit or to an internal coupling 64. The second valve 62 is a pump valve for controlling the coupling of the internal coupling 64 to either the atmospheric pressure or to the pump. Thus, in combination, an expression kit may be connected to the valve unit of the other expression kit, to atmospheric pressure or to the pump.

The controller 50 for example comprises a cyclic waveform generator for controlling the pump and for controlling the valve arrangement.

For the implementation using two valves 60, 62 as shown, the cyclic valve waveform generator in fact generates separate valve control signals. One signal is for the coupling valve 60 and the other is for the pump valve 62.

When venting from one kit to the other, the last part of the pressure leveling function is slower than the first part because of the pressure difference becoming smaller. To keep a constant slope, the pump therefore could help to make the second part as steep as the first part by already switching it on a little bit earlier and switching the valves accordingly, and hence not delay the use of the pump until the pressure is totally equal between the kits. This will however compromise the amount of energy that is gained.

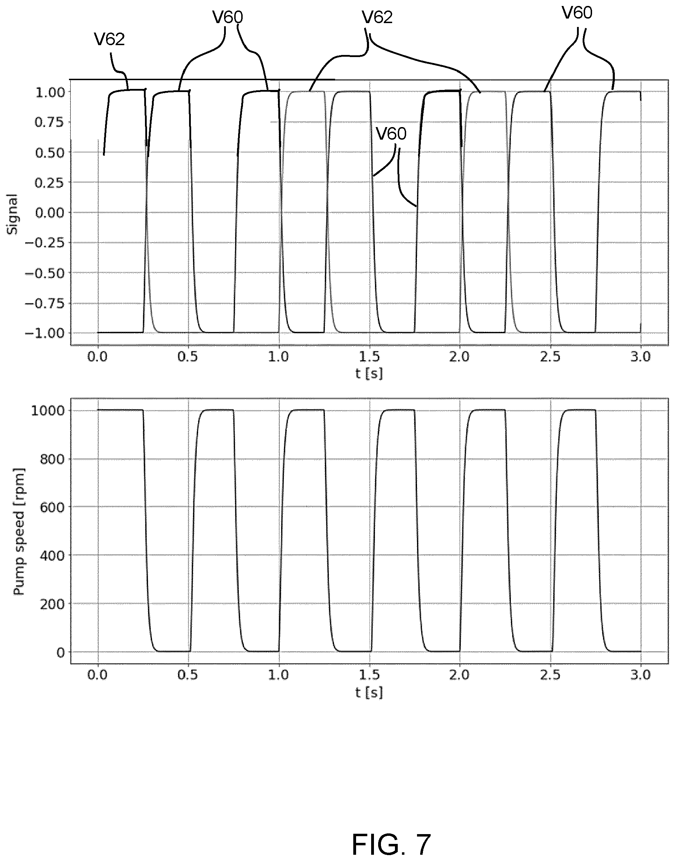

Figure 7 shows in the top graph the control signals for the valves 60, 62 (as V60 and V62). The bottom graph shows the pump control signal.

As shown, the pump is cycled on and off with a 50% duty cycle (because it is on only for two of the four cycles).

The first valve 60 is on for two of the four phases (which is when the expression kits are connected together). This coincides with the pump being turned off.

The valve 62 is only on (which is when the expression kit connects to the ambient surroundings) for one of the four cycles, when the connected expression kit is being vented to the ambient surroundings, e.g. phase 3 for the first expression kit 30.

The operation of the pump with 50% duty cycle (or slightly higher to ensure correct valve position or provide a smoother pressure profile, e.g. 50% to 55%) saves power.

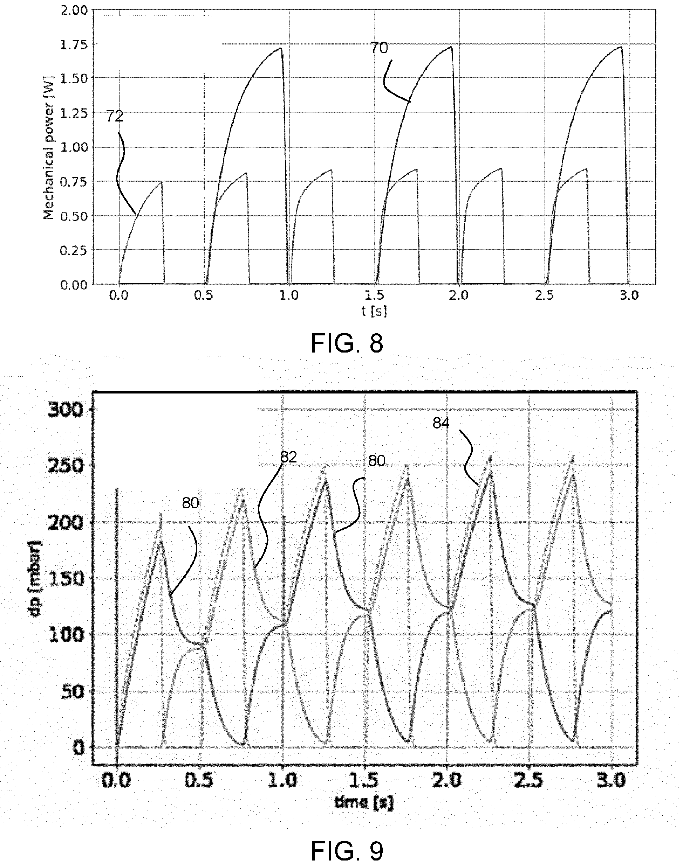

Figure 8 shows the mechanical power over time for a conventional use of a pair of independent expression kits (plot 70) and for the approach of the invention (plot 72).

The difference in pump fluid power (*P*=QΔ*P* - the area under the graph) is clear. For the conventional case, plot 70 is the total power of both pumps combined. Approximately half the energy is needed by the arrangement of the invention to obtain a similar performance compared to a standard double breast pump.

Figure 9 shows the cavity pressure 80 over time for one expression kit and the cavity pressure 82 over time for the other expression kit based on a simulation. Plot 84 is the pump pressure.

The described system enables a similar performance to be obtained as for two independent breast pumps but with around half of the energy consumption and one pump instead of two. Noise reduction is also achieved by reduced running times. Alternating suction is provided for two expression kits (i.e. two cups) and in particular using the vacuum on one side at the end of each cycle to already create part of the vacuum on the other side.

The use of a pair of three way valves is explained above. However, the ambient air venting function may use a mechanically controlled valve, so that the structure may differ from a standard three way valve.

The venting of the pressure from an expression kit to the ambient surroundings may be implemented through a flow restriction, to create a more comfortable pressure pattern. For example the abrupt changes in gradient seen at around 90 mbar (9 kPa) in Figure 9 may be smoothed. This may also be achieved by controlling the timing of switching the pump and valves as mentioned above.

The pump may simply be designed to deliver a single pressure level (a negative pressure level below atmospheric pressure), and the pressure will decrease towards that pressure level over time when it is connected to an expression kit. However, the pump may be controllable to deliver different pressure levels, either as different static pressure levels for different modes (e.g. stimulation and expression) or to control a pressure waveform over time more accurately.

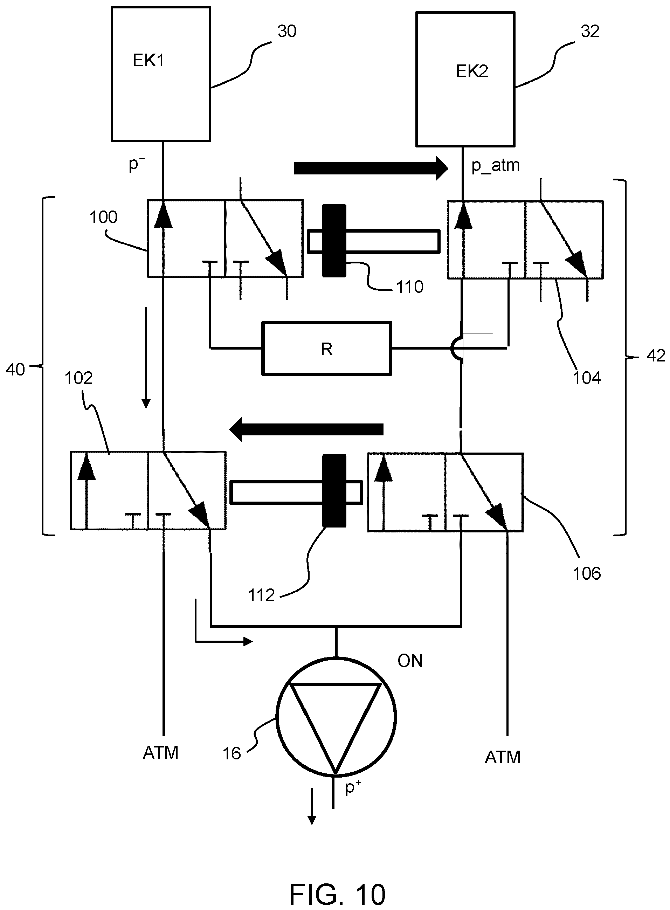

It is mentioned above that the use of pairs of three-port valves can simplify the control requirements as the number components as well as the energy consumed by the solenoids. An example is shown to explain this in Figures 10 to 12.

As shown in Figure 10, the first valve unit 40 is connected between the first kit 30 and the pump 16 and the second valve unit 42 is connected between the second kit 32 and the pump 16, as explained above.

The first valve unit 40 comprises a first three-port valve 100 and a second three-port valve 102. Each three port valve has two actuation positions, providing two possible connection paths between the three ports i.e. the three fluid connections to the valve.

The second valve unit 42 comprises a first three-port valve 104 and a second three-port valve 106. Each three port valve again has two actuation positions, providing two possible connection paths between the three ports.

As in the more schematic example above, the first valve unit 40 is controllable to connect the first kit to a selected one of the pump, the ambient pressure and the second kit, and the second valve unit 42 is controllable to connect the second kit to a selected one of the pump, the ambient pressure and the first kit.

A first actuator 110, e.g. solenoid, is provided for actuating the first three-port valve 100 of the first valve unit and (synchronously and hence non-independently) the first three-port valve 104 of the second valve unit, and a second actuator 112, e.g. solenoid, is provided for actuating the second three-port valve 102 of the first valve unit and (synchronously and hence non-independently) the second three-port valve 106 of the second valve unit.

The first three-port valve 100 of the first valve unit has a connection to the first kit 30, a connection to the first three-port valve 104 of the second valve unit and a connection to the second three-port valve 102 of the first valve unit. These are the three ports of the three-port valve.

The second three-port valve 102 of the first valve unit has a connection to the ambient pressure, a connection to the pump 16 and a connection to the first three-port valve 100 of the first valve unit. These are the three ports of the three-port valve.

The first three-port valve 104 of the second valve unit has a connection to the second kit 32, a connection to the first three-port valve 100 of the first valve unit and a connection to the second three-port valve 106 of the second valve unit. These are the three ports of the three-port valve.

The second three-port valve 106 of the second valve unit has a connection to the ambient pressure, a connection to the pump 16 and a connection to the first three-port valve 104 of the second valve unit. These are the three ports of the three-port valve.

Figure 10 shows the first phase with a negative pressure coupled to the first kit 30 and the second kit vented to the ambient pressure. To achieve these connections, the first actuator 110 is driven to use the left connection option for the three-port valves 100,104 and the second actuator 112 is driven to use the right connection option for the three port valves 102, 106.

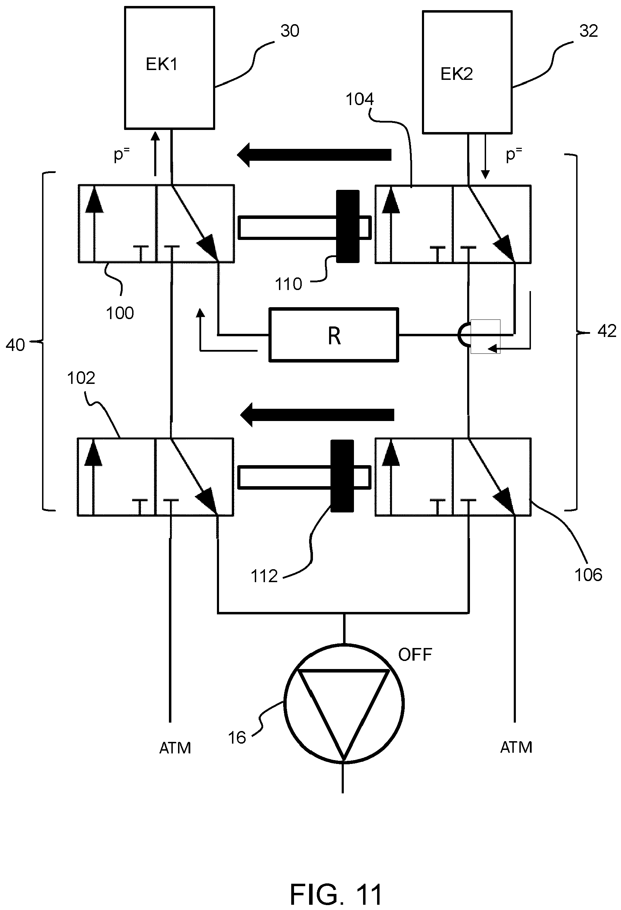

Figure 11 shows the second phase with the first and second kits coupled together (and the pump isolated from both kits with no venting to ambient pressure). To achieve these connections, the first actuator 110 is driven to use the right connection option for the three-port valves 100,104 and the second actuator 112 is also driven to use the right connection option for the three port valves 102, 106.

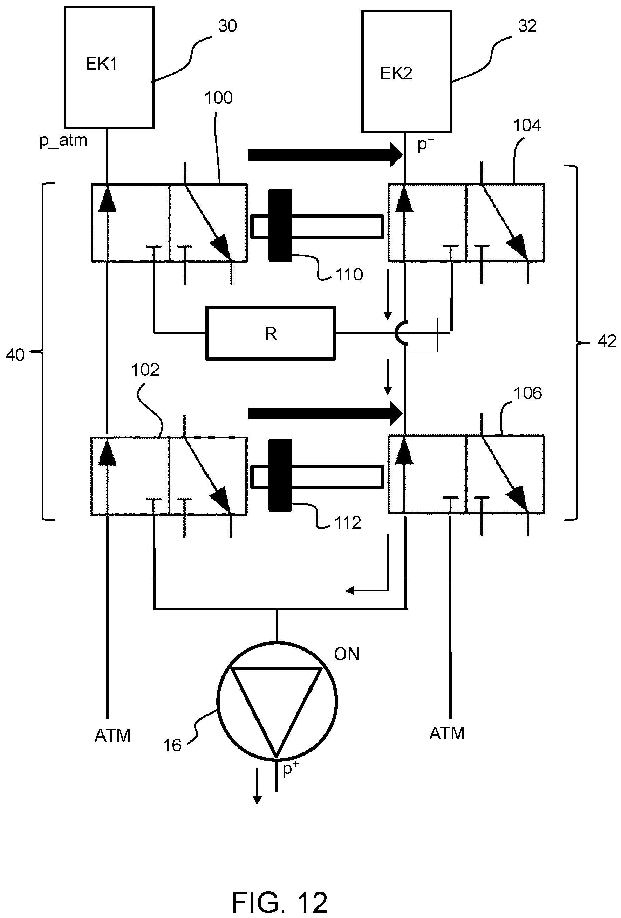

Figure 12 shows the third phase with a negative pressure coupled to the second kit and the first kit vented to the ambient pressure. To achieve these connections, the first actuator 110 is driven to use the left connection option for the three-port valves 100,104 and the second actuator 112 is also driven to use the left connection option for the three port valves 102, 106.

The fourth phase is the same as the second phase.

The example above is based on the use of two expression kits.

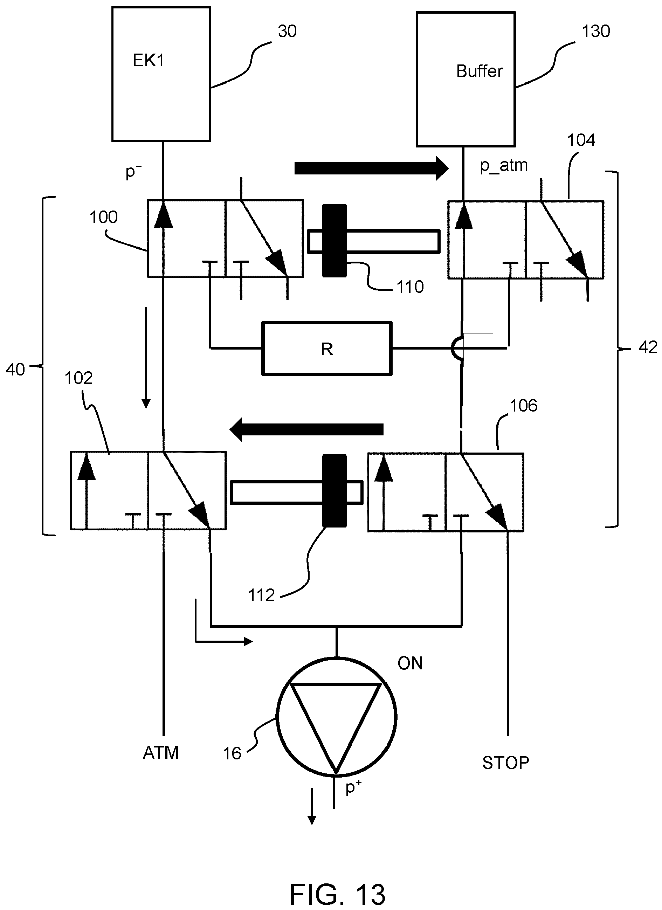

A first example based on the use of a single expression kit 30 (EK1) and a buffer 130 will now be explained with reference to Figures 13 to 17.

As in the example above, the first valve unit 40 comprise a first three-port valves 100 and a second three port-valve coupled together, and the second valve unit 42 comprises a first three-port valve 104 and a second three-port valve 106 coupled together. Each three port valve again has two actuation positions, providing two possible connection paths between the three ports.

The first valve unit 40 is controllable to connect the first kit to a selected one of the pump, the ambient pressure and the buffer. It connects the second valve unit 42 to the buffer.

In this example, the second valve unit 42 is controllable to connect the buffer 136 to a selected one of the pump, a pressure stop and the first kit (via valve unit 40). However, as will clear from the description below, the buffer is connected to the pump when it is turned off, and this is used as another stop.

A first actuator 110, e.g. solenoid, is again provided for actuating the first three-port valve 100 of the first valve unit and (synchronously and hence non-independently) the first three-port valve 104 of the second valve unit, and a second actuator 112, e.g. solenoid, is provided for actuating the second three-port valve 102 of the first valve unit and (synchronously and hence non-independently) the second three-port valve 106 of the second valve unit.

The first three-port valve 100 of the first valve unit has a connection to the first kit 30, a connection to the first three-port valve 104 of the second valve unit and a connection to the second three-port valve 102 of the first valve unit. These are the three ports of the three-port valve.

The second three-port valve 102 of the first valve unit has a connection to the ambient pressure (ATM), a connection to the pump 16 and a connection to the first three-port valve 100 of the first valve unit. These are the three ports of the three-port valve.

The first three-port valve 104 of the second valve unit has a connection to the buffer 130, a connection to the first three-port valve 100 of the first valve unit and a connection to the second three-port valve 106 of the second valve unit. These are the three ports of the three-port valve.

The second three-port valve 106 of the second valve unit has a connection to a stop, a connection to the pump 16 and a connection to the first three-port valve 104 of the second valve unit. These are the three ports of the three-port valve.

Figure 13 shows the beginning of a start-up phase. The buffer pressure is initially the ambient pressure p_atm (or it may be an under pressure that is still present from a previous pumping session.

The first kit is connected to the pump, which is on, and the buffer is connected to the stop (though both valves 104, 106).

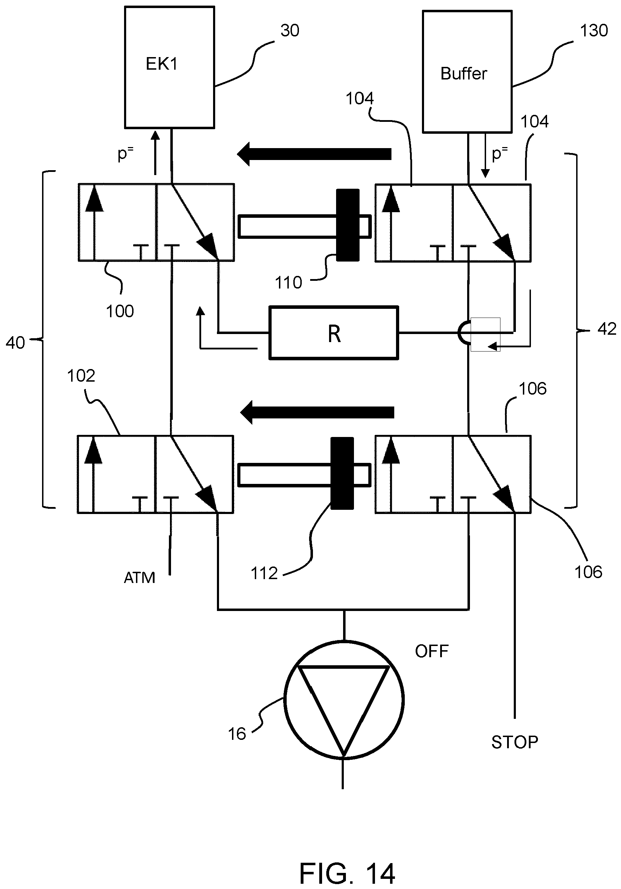

Figure 14 shows the pressure equalization to pressurize the buffer. The first kit is connected to the buffer and the pump is off. The resulting buffer pressure will depend on the buffer volume and expression kit volume (i.e. the ratio) in combination with the previous pressures in the buffer and expression kit, following the Ideal gas law. A start-up behavior arises because the buffer starts at ambient pressure (or a previously present pressure).

Figures 13 and 14 are steps of an initial pressure setting procedure.

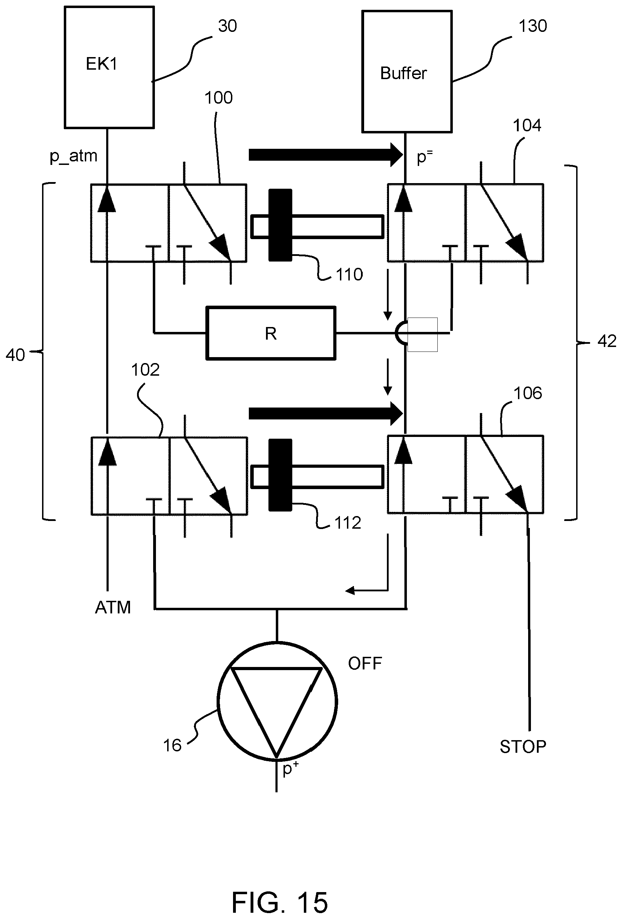

Figure 15 shows the first expression kit vented to the ambient pressure ATM by the first valve unit 40 and the buffer connected to the pump. However, the pump is off, an no air leakage is allowed through the pump (or a valve may be added to ensure this such as a passive one-way valve). Thus, the buffer is effectively connected to a stop so the buffer pressure is maintained.

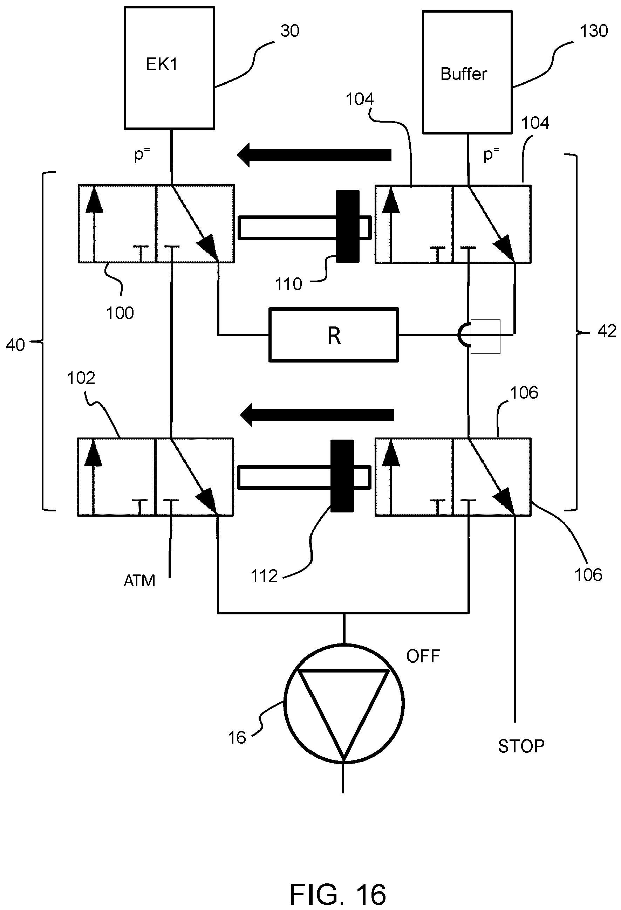

Figure 16 shows the first expression kit connected to the buffer, with the pump off. This is the same pressure equalization as Figure 14.

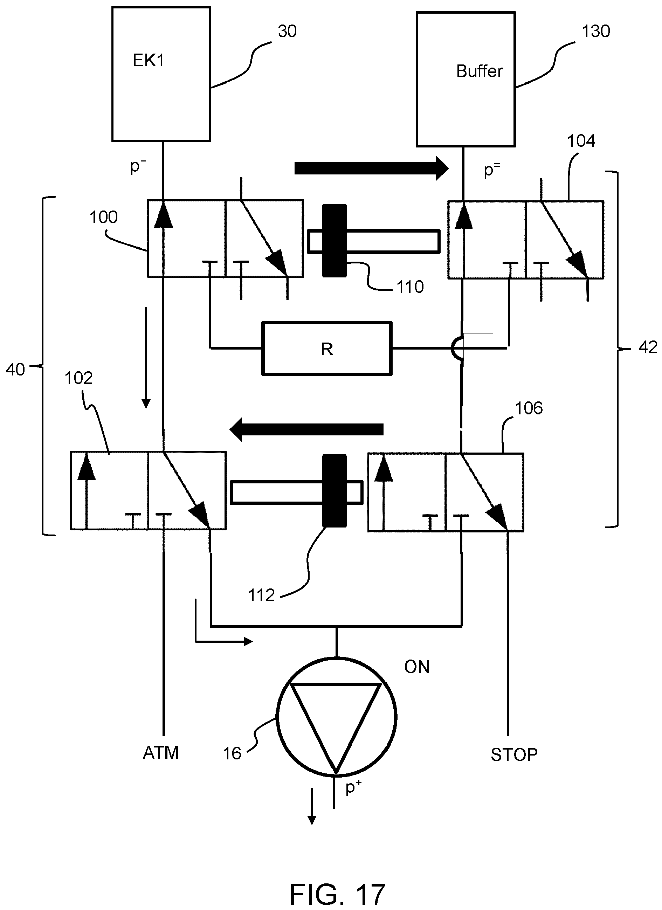

Figure 17 shows the expression kit connected to the pump again by the first valve unit and the buffer connected to the stop by the second valve unit. This is the same as Figure 13.

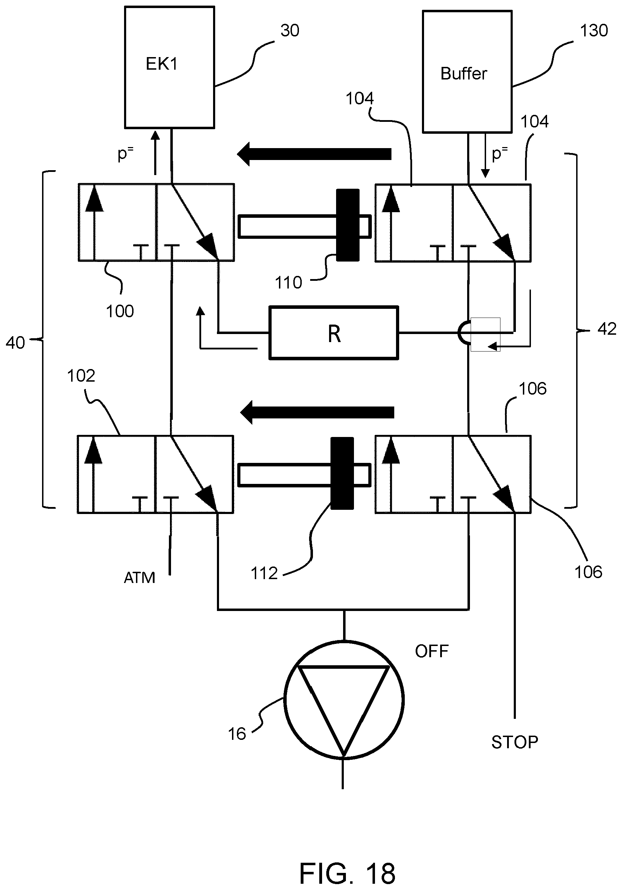

Figure 18 shows the pressure equalization step, again the same as Figure 14..

Figures 15 to 18 shows the four cycles of the expression cycle, and they are repeated sequentially.

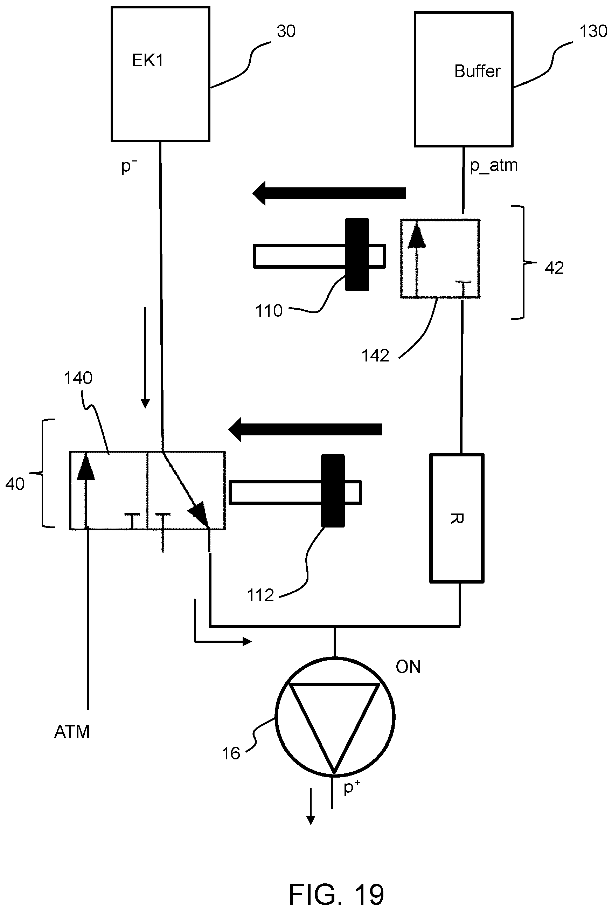

A second example based on the use of a single expression kit 30 (EK1) and a buffer 130 will now be explained with reference to Figures 19 to 24.

The first valve unit 40 in this case comprises a single three-way valve 140 with connections to the ambient pressure ATM, the first expression kit 30 and the pump 16.

The second valve unit 42 is a two-way valve 142 which either connects the buffer 130 to the pump or isolates the buffer by connecting it to a pressure stop. As in the example above, when the buffer is connected to the pump, the pump is off, so this in fact is used to provide a connection between the buffer and the expression kit.

Figure 19 shows a first initial configuration. The buffer starts at atmospheric pressure as in the example above (or at a previously present pressure). The expression kit 30 is coupled to the pump by the first valve unit and the buffer is isolated by the second valve unit.

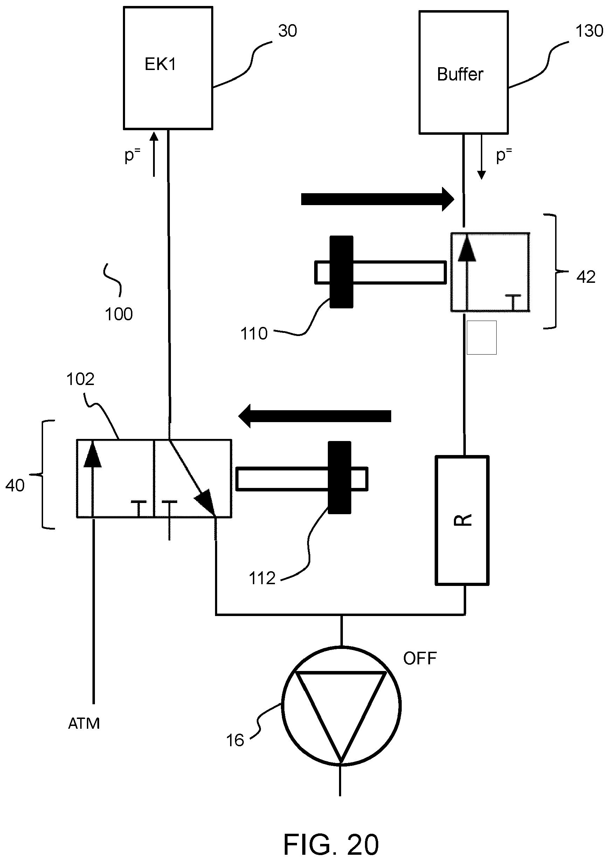

Figure 20 shows the pressure equalizing, with the expression kit coupled to the buffer (because they both connect to the pump) and the pump is turned off. As in the example above, no air leakage is permitted through the pump, or the pump may be run at a low speed to prevent inflow. An extra valve may also be added for this purpose.

Figures 19 and 20 are steps of an initial pressure setting procedure.

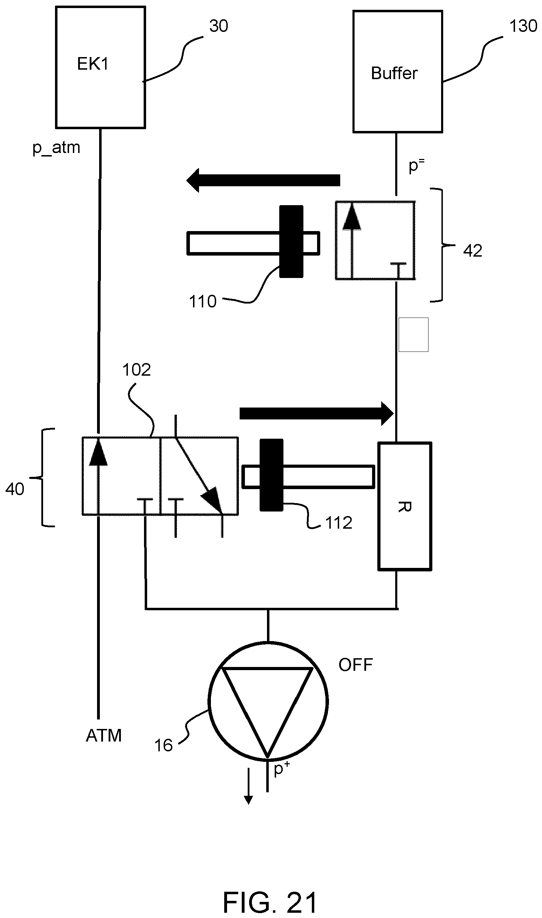

Figure 21 shows the venting of the pressure of the expression kit to reset the expression kit, by connecting the expression kit to atmospheric pressure using the first valve unit. The buffer is isolated by the second valve unit. The pump is off but it could also be used in a (lower) setting to pump more out of the buffer. In that way, a less strong pump is needed giving a cost saving. The pump may run during all the cycles giving better noise performance.

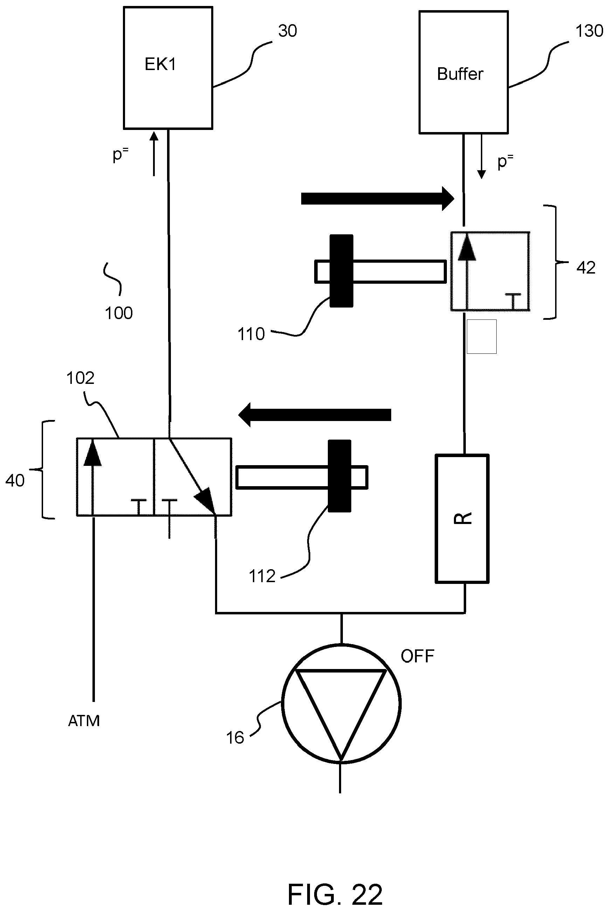

Figure 22 shows the pressure equalizing, equivalent to Figure 20.

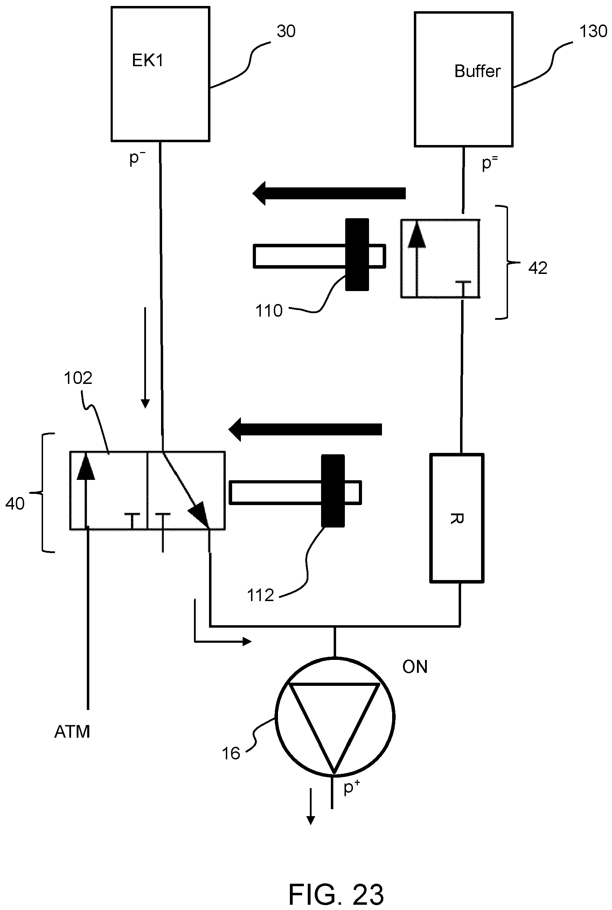

Figure 23 shows the expression kit connected to the pump and the pump turned on, to implement the pressure cycle of the expression kit.

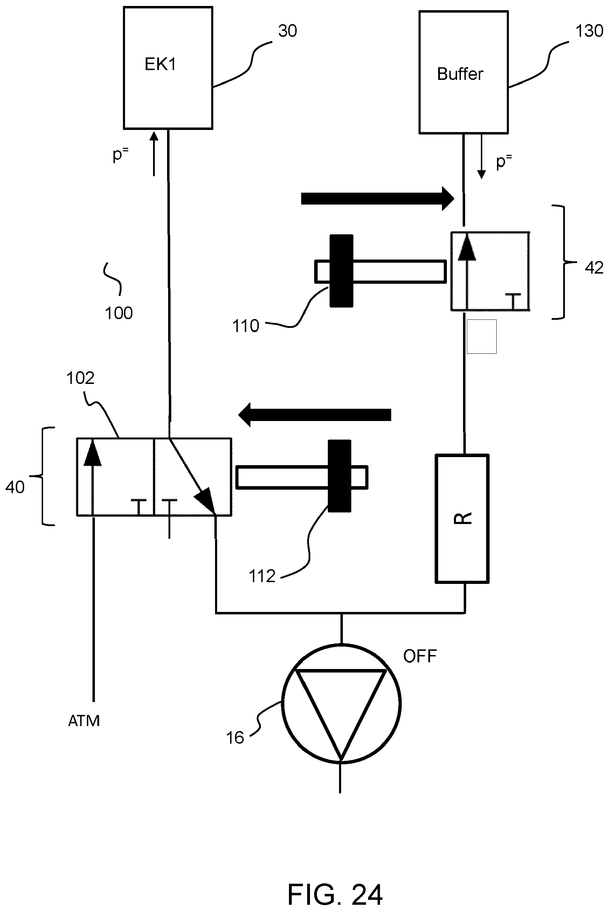

Figure 24 shows the pump turned off and pressure equalizing again performed.

Figures 21 to 24 show the four cycles of the expression cycle, and they are repeated sequentially.

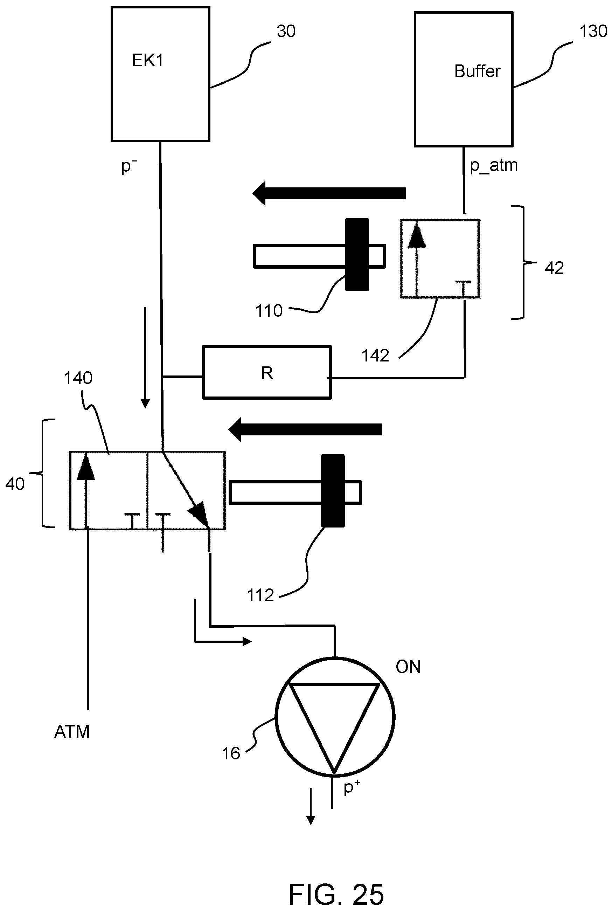

A third example based on the use of a single expression kit 30 (EK1) and a buffer 130 will now be explained with reference to Figures 25 to 30.

The first valve unit 40 in this case comprises a single three-way valve 140 with connections to the ambient pressure ATM, the first expression kit 30 and the pump 16.

The second valve unit 42 is a two-way valve 142 which either connects the buffer 130 to the first valve unit 40 or isolates the buffer by connecting it to a pressure stop. The connection to the first valve unit enables the buffer and first expression kit to be connected together (because they both connect to the same port of the first valve unit 40).

Figure 25 shows a first initial configuration. The buffer starts at atmospheric pressure as in the example above (or at a previously present pressure). The expression kit 30 is coupled to the pump by the first valve unit and the buffer is isolated by the second valve unit.

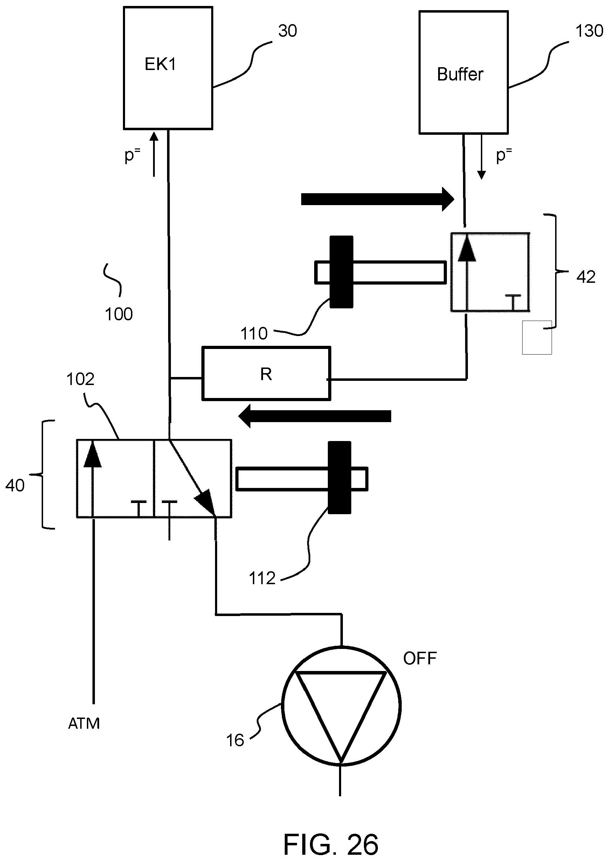

Figure 26 shows the pressure equalizing, with the expression kit coupled to the buffer (because they both connect to the first valve unit and then to the pump). The pump is turned off. As in the example above, no air leakage is permitted through the pump, or the pump may be run at a low speed to prevent inflow. An extra valve may again be added for this purpose.

Figures 25 and 26 are steps of an initial pressure setting procedure.

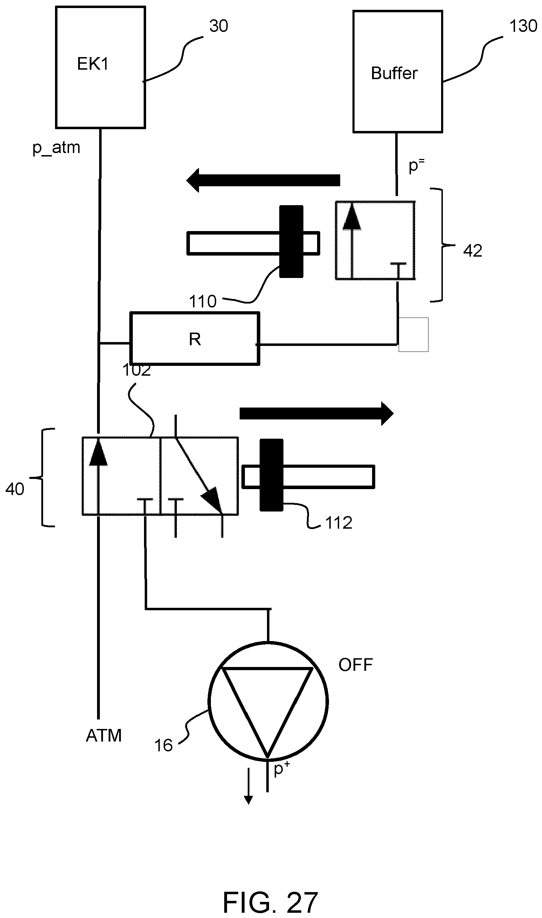

Figure 27 shows the venting of the pressure of the expression kit to reset the expression kit, by connecting the expression kit to atmospheric pressure using the first valve unit 40. The buffer is isolated by the second valve unit 42. The pump is off.

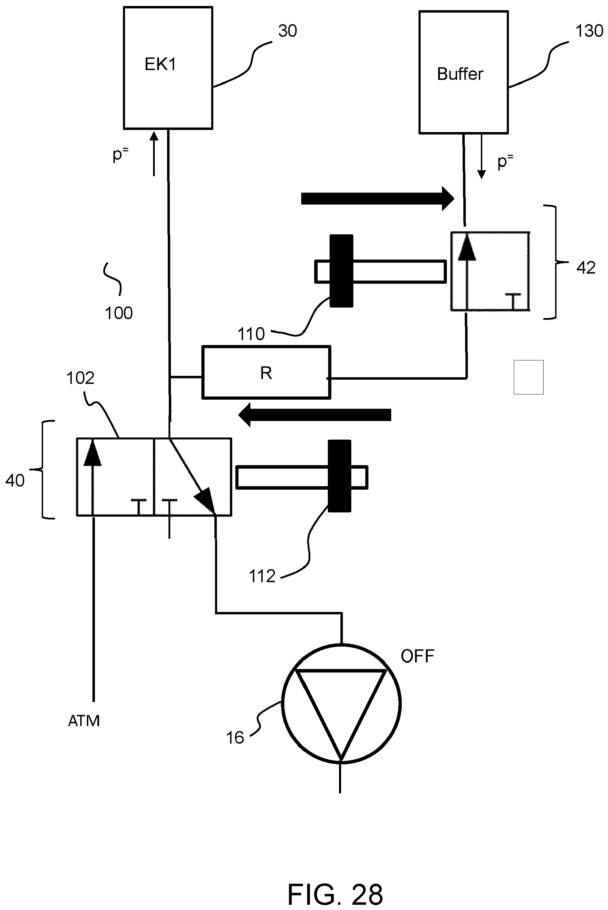

Figure 28 shows the pressure equalizing, equivalent to Figure 26.

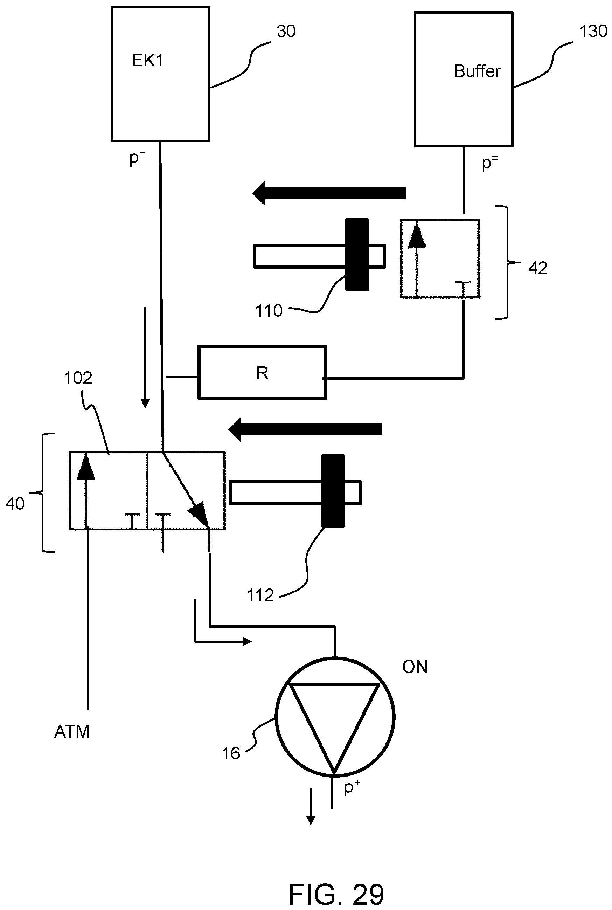

Figure 29 shows the expression kit 30 connected to the pump and the pump turned on, to implement the pressure cycle of the expression kit. The buffer is isolated by the second valve unit 42.

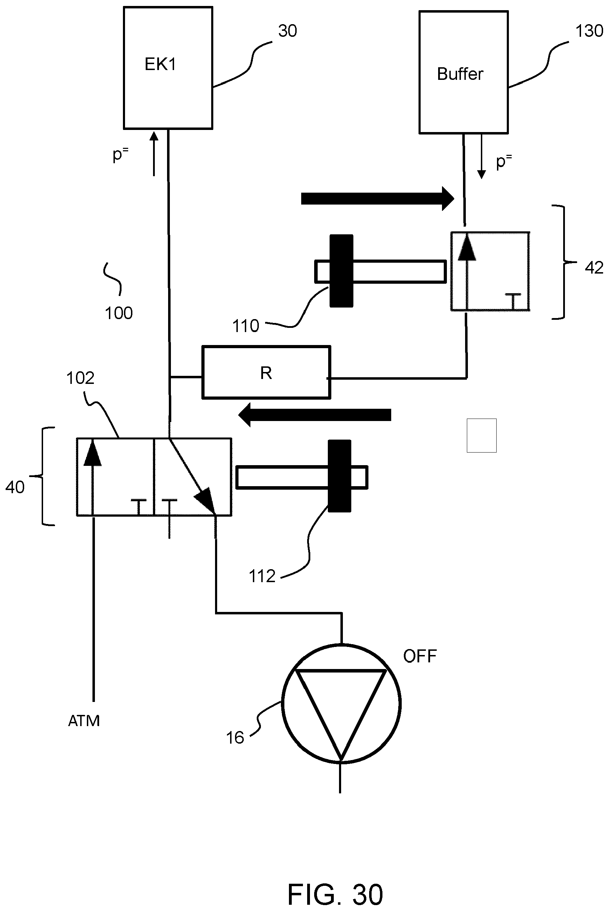

Figure 30 shows the pump turned off and pressure equalizing again performed by switching the second valve unit.

Figures 27 to 30 show the four cycles of the expression cycle, and they are repeated sequentially.

The invention also provides a method of controlling the breast pump system for the non-therapeutic expression of milk.

The method comprises:
controlling a single pump to generate a negative pressure;
controlling the single pump and the valve arrangement to apply a negative pressure to the first kit and equalize a pressure level between the first kit and second kit or buffer, by:
   controlling a first valve unit 40 connected between the first kit and the pump and a second valve unit 42 connected between the second kit or buffer and the pump or the first valve unit,
   wherein the first and second valve units are controlled to connect:
      the first kit to a selected one of the pump, the ambient pressure and the second kit or buffer; and
      the second kit or buffer to the first kit,
      and wherein the method comprises controlling each of the first and second valve units (40, 42) with a single respective valve actuator.

A cyclic pressure waveform may be applied to the first kit (and second kit if present) in the form of an expression waveform. However, the same approach may be applied to a stimulation waveform.

When there are cyclic expression and stimulation modes, they for example have different cyclic frequency and different maximum under-pressure (i.e. vacuum) levels. The expression mode for example cycles between a baseline vacuum and a maximum vacuum (i.e. a maximum under-pressure) "Max_Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline_Vac = atmospheric pressure
Max_Vac = 300 mbar (30 kPa) below atmospheric pressure
TTV = 0.85 s
DI = 0.3 s
TTA = 0.05 s
DO = 0.35 s
This gives a total cycle time T_cycle of 1.55s.

These values are simply by way of example. The maximum under-pressure may be higher, for example 350 mbar (35 kPa) and the durations may differ. The cycle time is generally of the order of 1 to 2 seconds, typically 1 second (1 Hz).

The stimulation mode for example has a faster frequency hence with a shorter cycle time of around 0.5 seconds (2 Hz), and a lower vacuum, for example a maximum under-pressure level of 150 mbar (15 kPa).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system comprising:
a first kit (30); and
a second kit (32) or a buffer (130),
a pump (16) for delivering a negative pressure;
a valve arrangement (40,42) which selectively provides an interconnection between the pump, the ambient pressure, the first kit and the second kit or buffer; and
a controller for controlling the valve arrangement,
wherein the valve arrangement comprises:
a first valve unit (40) connected between the first kit (30) and the pump (16) and a second valve unit (42) connected between the second kit (32) or buffer and the pump (16) or the first valve unit (40), wherein the first and second valve units (40,42) are controllable to connect the first kit to a selected one of the pump, the ambient pressure and the second kit or buffer,
and wherein the first and second valve units (40, 42) each comprise a single valve actuator.

2. The breast pump system of claim 1, wherein the second kit (32) or buffer comprises a buffer (130), the first valve unit (40) comprises a three-port valve (140) which couples the first kit, the ambient pressure and the pump, and the second valve unit (42) couples the buffer to the first valve unit or to a pressure stop.

3. The breast pump system of claim 1, wherein the second kit (32) or buffer comprises a buffer, the first valve unit (40) comprises a pair of three-port valves which couple the first kit, the ambient pressure, the buffer and the pump, and the second valve unit comprises a pair of three port valves which couple the buffer, the first kit, the pump and a pressure stop.

4. The breast pump system of claim 1, wherein the second kit or buffer comprises a second kit, and wherein the first and second valve units (40, 42) each comprise a pair of three-port valves coupled together, thereby having four external ports.

5. The breast pump system of claim 4, wherein the controller is adapted to control the valve arrangement to couple:
the first kit to the pump and the second kit or buffer to the ambient pressure in a first configuration;
the first kit and the second kit together in a second configuration to equalize a pressure level between the first kit and second kit; and
the second kit to the pump and the first kit to the ambient pressure in a third configuration.

6. The breast pump system of claim 5, wherein the controller comprises a cyclic pump waveform generator for controlling the pump and for controlling the valve arrangement.

7. The breast pump system of claim 6, wherein the cyclic waveform generator generates a cyclic pump waveform and a cyclic valve waveform, thereby to implement a cycle with four phases in sequence:
a first phase comprising the first configuration and with the pump generating a negative pressure;
a second phase comprising the second configuration;
a third phase comprising the third configuration and with the pump generating a negative pressure;
a fourth phase comprising the second configuration.

8. The breast pump system of claim 7, wherein the pump is turned off in the second and fourth phases.

9. The breast pump system of any one of claims 4 to 8, comprising a first actuator for actuating a first three-port valve of the first valve unit and a first three-port valve of the second valve unit, and a second actuator for actuating a second three-port valve of the first valve unit and a second three-port valve of the second valve unit.

10. The breast pump system of claim 9, wherein:
the first three-port valve of the first valve unit has a connection to the first kit, a connection to the first three-port valve of the second valve unit and a connection to the second three-port valve of the first valve unit;
the second three-port valve of the first valve unit has a connection to the ambient pressure, a connection to the pump and a connection to the first three-port valve of the first valve unit;
the first three-port valve of the second valve unit has a connection to the second kit or buffer, a connection to the first three-port valve of the first valve unit and a connection to the second three-port valve of the second valve unit; and
the second three-port valve of the second valve unit has a connection to the ambient pressure, a connection to the pump and a connection to the first three-port valve of the second valve unit.

11. The breast pump system of any one of claims 1 to 10, wherein each kit comprises a suction cup, comprising two air chambers separated by a flexible movable membrane.

12. A method of controlling a breast pump system for the non-therapeutic expression of milk, wherein the breast pump system has a first kit (30), a second kit (32) or a buffer, a pump (16) for delivering a negative pressure and a valve arrangement which selectively provides an interconnection between the pump, the ambient pressure, the first kit and the second kit or buffer,
wherein the method comprises:
controlling a single pump to generate a negative pressure;
controlling the single pump and the valve arrangement to apply a negative pressure to the first kit and equalize a pressure level between the first kit and second kit or buffer, by:
controlling a first valve unit (40) connected between the first kit (30) and the pump (16) and a second valve unit (42) connected between the second kit (32) or buffer and the pump (16) or the first valve unit (40),
wherein the first and second valve units (40,42) are controlled to connect the first kit to a selected one of the pump, the ambient pressure and the second kit or buffer,
and wherein the method comprises controlling each of the first and second valve units (40, 42) with a single respective valve actuator.

13. The method of claim 12, comprising controlling the pump to generate the negative pressure with a cyclic pump waveform and controlling the valve arrangement to apply the negative pressure to the first kit and to a second kit in an alternating sequence using a cyclic valve waveform.

14. The method of claim 13, comprising implementing a cycle with four phases in sequence:
a first phase with a negative pressure coupled to the first kit and the second kit vented to the ambient pressure;
a second phase with the first kit and second kit coupled together
a third phase with a negative pressure coupled to the second kit and the first kit vented to the ambient pressure; and
a fourth phase with the first kit and second kit coupled together.

15. A computer program comprising computer program code means which is adapted, when said program is run on a controller of a breast pump system, to implement the method of any one of claims 12 to 14.
